# EUROPEAN PATENT SPECIFICATION

(11) **EP 3 880 169 B1**
(45) Date of publication and mention of the grant of the patent: **23.07.2025**
(21) Application number: 19808894.0
(22) Date of filing: 12.11.2019
(51) Int. Cl.: A61K 9/00, A61K 9/12, A61K 31/138, A61K 31/439, A61M 15/00, A61P 11/08

(54) **UMECLIDINIUM AND VILANTEROL FORMULATION AND INHALER**
UMECLIDINIUM UND VILANTEROL FORMULIERUNG UND INHALATOR
FORMULATION D'UMECLIDINIUM ET VILANTEROL ET INHALATEUR

(30) Priority: 12.11.2018 US 201862758758 P
(43) Date of publication of application: 22.09.2021
(73) Proprietor: Kindeva Drug Delivery L.P., St. Paul, MN 55170 (US)
(72) Inventor: DEXTER, Sarah J., Oakville, Ontario L64 3V2 (CA); LISTER, James T., Bracknell Berkshire RG12 8HT (GB)
(74) Representative: Vossius & Partner Patentanwälte Rechtsanwälte mbB
(86) International application number: PCT/IB2019/059716
(87) International publication number: WO 2020/100040

(56) References cited:
- WO-A1-2012/158166
- WO-A1-2013/153146
- WO-A1-2014/155134
- WO-A1-2015/179511
- WO-A1-2017/033032
- WO-A1-2018/051131
- DONALD P TASHKIN ET AL: "Combination bronchodilator therapy in the management of chronic obstructive pulmonary disease", RESPIRATORY RESEARCH, BIOMED CENTRAL LTD., LONDON, GB, vol. 14, no. 1, 8 May 2013 (2013-05-08), pages 49, XP021147771, ISSN: 1465-9921, DOI: 10.1186/1465-9921-14-49

## Description

### FIELD

The present disclosure generally relates to formulations used for, as an example, inhaled dosage forms, as well as aerosol canisters, inhalers, such as metered dose inhalers, containing the same. In particular, the present disclosure relates to formulations including umeclidinium and vilanterol.

### BACKGROUND

Currently, dry-powder inhalers (DPIs) containing umeclidinium bromide and vilanterol trifenatate are commercially available. These include GlaxoSmithKline's Anoro^{®} Ellipta^{®} DPIs.
WO 2013/153146 A1 discloses aggregate particles comprising nanoparticulate drug particles of umeclidinium bromide.
WO 2014/155134 A1 describes the use of a stearate in an inhalable formulation for improving dispensed dosing reproducibility of the inhalable formulation from an automated powder filling apparatus.
WO 2017/033032 A1 provides a pharmaceutical composition for inhalation comprising a first active agent and a second active agent, wherein the aerodynamic particle size distribution of the first and second active agents relative to their delivered doses is substantially the same.

### Summary

Pressurized metered dose inhalers (pMDIs) containing umeclidinium and vilanterol may have several advantages over DPIs. For example, controlling the stability of the micronized medicament so that it delivers a consistent dose to a patient, and, in some instances, pMDI manufacture may be done less expensively than a DPI product.

The present invention is defined in the appended claims.

Other features and aspects of the present disclosure will become apparent by consideration of the detailed description.

### DETAILED DESCRIPTION

Throughout this disclosure, singular forms such as "a," "an," and "the" are often used for convenience; singular forms are meant to include the plural unless the singular alone is explicitly specified or is clearly indicated by the context. Numerical ranges, for example "between x and y" or "from x to y", include the endpoint values of x and y.

Some terms used in this application have special meanings, as defined herein. All other terms will be known to the skilled artisan and are to be afforded the meaning that a person of skill in the art at the time of the invention would have given them.

Elements in this specification that are referred to as "common" and "commonly used" and similar descriptors should be understood to be common within the context of the compositions, articles, such as inhalers and metered dose inhalers, and methods of this disclosure; this terminology is not used to mean that these features are present, much less common, in the prior art. Unless otherwise specified, only the Background section of this Application refers to the prior art.

The "particle size" of a single particle is the size of the smallest hypothetical hollow sphere that could encapsulate the particle.

The "mass median diameter" or MMD of a plurality of particles refers to the value for a particle diameter at which 50% of the mass of particles in the plurality of particles have a particle size smaller than the value and 50% of the mass of particles in the plurality of particle have a particle size greater than the value.

The "canister size" of a plurality of particles refers to the mass mean diameter of the plurality of particles when the formulation is prepared.

The "ex-actuator sizeʺʺ of a plurality of particles refers to the mass median aerodynamic diameter (or MMAD) of the plurality of particles after the plurality of particles has passed through the actuator of an inhaler, such as a metered dose inhaler, as measured by the procedure described in the United States Pharmacopeia <601>.

When the concentration of umeclidinium is discussed in this application, for convenience it is referred to in terms of the concentration of the form of umeclidinium that is most commonly used in this disclosure, that is, umeclidinium bromide. It should therefore be understood that if another form or salt of umeclidinium is used, the concentration of that other form or salt should be calculated on a basis relative to umeclidinium bromide. A person of ordinary skill in the relevant arts can easily perform this calculation by comparing the molecular weight of the form or salt of umeclidinium that is used to the molecular weight of umeclidinium bromide. When doses of umeclidinium are discussed, such as nominal product doses or measured delivered doses, they are given for umeclidinium base unless otherwise specified.

When the formulation concentration of vilanterol is discussed in this application, for convenience it is referred to in terms of the concentration of the form of vilanterol that is most commonly used in this disclosure, that is, vilanterol trifenatate, unless otherwise specified. It should therefore be understood that if another form or salt of vilanterol is used, the formulation concentration of that other form or salt should be calculated on a basis relative to vilanterol trifenatate. A person of ordinary skill in the relevant arts can easily perform this calculation by comparing the molecular weight of the form or salt of vilanterol that is used to the molecular weight of vilanterol trifenatate. When doses of vilanterol are discussed, such as nominal product doses or measured delivered doses, they are given for vilanterol base unless otherwise specified.

### Formulation

A pharmaceutical formulation comprises particulate umeclidinium. The umeclidinium can be a free base but can be in the form of one or more physiologically acceptable salts or solvates, such as umeclidinium bromide.

The umeclidinium, such as umeclidinium bromide, is in particulate form. The canister size of the particles of umeclidinium, such as umeclidinium bromide, can be any suitable canister size. Exemplary suitable canister sizes can be no less than 1 micrometer, no less than 1.5 micrometers, no less than 2 micrometers, no less than 2.5 micrometers, no less than 3 micrometers, no less than 3.5 micrometers, no less than 4 micrometers, or no less than 4.5 micrometers. Exemplary suitable canister sizes can also be no greater than 5 micrometers, no greater than 4.5 micrometers, no greater than 4.0 micrometers, no greater than 3.5 micrometers, no greater than 3.0 micrometers, no greater than 2.5 micrometers, no greater than 2.0 micrometers, or no greater than 1.5 micrometers. 1 micrometer to 5 micrometers is common. In embodiments the canister size may be between 2.0 and 4.0 micrometers. In embodiments the canister size may be between 2.0 and 3.0 micrometers.

The ex-actuator size of the umeclidinium particles, such as umeclidinium bromide particles, can be any suitable ex-actuator size. Exemplary suitable ex-actuator sizes can be no less than 1 micrometer no less than 1.5 micrometers, no less than 2 micrometers, no less than 2.5 micrometers, no less than 3 micrometers, no less than 3.5 micrometers, no less than 4 micrometers, or no less than 4.5 micrometers. Exemplary suitable ex-actuator sizes can also be no greater than 5 micrometers, no greater than 4.5 micrometers, no greater than 4.0 micrometers, no greater than 3.5 micrometers, no greater than 3.0 micrometers, no greater than 2.5 micrometers, no greater than 2.0 micrometers, or no greater than 1.5 micrometers. 1 micrometer to 5 micrometers is common. In embodiments the ex-actuator size may be between 2.0 and 4.0 micrometers. In embodiments the ex-actuator size may be between 2.5 and 3.5 micrometers.

The umeclidinium, such as umeclidinium bromide, can be present in any suitable concentration in the formulation. When the concentration of umeclidinium is expressed in terms of mg/g of formulation, then the concentration of umeclidinium can be no less than 0.1, no less than 0.2, no less than 0.3, no less than 0.4, no less than 0.5, no less than 0.6, no less than 0.7, no less than 0.8, no less than 0.9, no less than 1.0, no less than 1.5, or no less than 2.0. Also, on a mg/g basis, the concentration of umeclidinium can be no greater than 10.0, no greater than 8.0, no greater than 6.0, no greater than 5.0, no greater than 4.0, no greater than 3.0, no greater than 2.5, no greater than 2.2, or no greater than 2.0. Common concentrations are from 0.5 mg/g to 10.0 mg/g, such as 0.5 mg/g to 5.0 mg/g. In an embodiment, a concentration of about 1.3 mg/g is employed. In an additional embodiment, a concentration of about 1.6 mg/g is employed. In yet another embodiment, a concentration of 3.3 mg/g is employed.

The composition also comprises vilanterol trifenatate. The vilanterol trifenatate is also in particulate form. The canister size of the particles of vilanterol, such as vilanterol trifenatate, can be any suitable canister size. Exemplary suitable canister sizes can be no less than 1 micrometer no less than 1.5 micrometers, no less than 2 micrometers, no less than 2.5 micrometers, no less than 3 micrometers, no less than 3.5 micrometers, no less than 4 micrometers, or no less than 4.5 micrometers. Exemplary suitable canister sizes can also be no greater than 5 micrometers, no greater than 4.5 micrometers, no greater than 4.0 micrometers, no greater than 3.5 micrometers, no greater than 3.0 micrometers, no greater than 2.5 micrometers, no greater than 2.0 micrometers, or no greater than 1.5 micrometers. 1 micrometer to 5 micrometers is common. In embodiments the canister size may be between 3.0 and 4.5 micrometers. In embodiments the canister size may be between 1.0 and 2.0 micrometers.

The ex-actuator size of the vilanterol trifenatate particles can be any suitable ex-actuator size. Exemplary suitable ex-actuator sizes can be no less than 1 micrometer no less than 1.5 micrometers, no less than 2 micrometers, no less than 2.5 micrometers, no less than 3 micrometers, no less than 3.5 micrometers, no less than 4 micrometers, or no less than 4.5 micrometers. Exemplary suitable ex-actuator sizes can also be no greater than 5 micrometers, no greater than 4.5 micrometers, no greater than 4.0 micrometers, no greater than 3.5 micrometers, no greater than 3.0 micrometers, no greater than 2.5 micrometers, no greater than 2.0 micrometers, or no greater than 1.5 micrometers. 1 micrometer to 5 micrometers is common. In embodiments the ex-actuator size may be between 1.0 and 4.0 micrometers. In embodiments the ex-actuator size may be between 1.5 and 2.5 micrometers.

The vilanterol can be used in any suitable concentration. On a mg/g basis, exemplary concentrations are no less than 0.05, no less than 0.10, no less than 0.15, no less than 0.20, no less than 0.25, no less than 0.30, no less than 0.35, no less than 0.40, no less than 0.45, or no less than 0.5. Exemplary concentrations are also no greater than 2.0, no greater than 1.9, no greater than 1.8, no greater than 1.7, no greater than 1.6, no greater than 1.5, no greater than 1.4, no greater than 1.3, no greater than 1.2, no greater than 1.1, no greater than 1.0, no greater than 0.9, no greater than 0.8, no greater than 0.7, no greater than 0.6, or no greater than 0.5. Common concentrations are from 0.2 mg/g to 5.0 mg/g, such as 0.4 mg/g to 2.5 mg/g. In an embodiment, a concentration of about 0.7 mg/g is employed. In an additional embodiment, a concentration of about 0.9 mg/g is employed. In yet another embodiment, a concentration of 1.8 mg/g is employed.

In some embodiments, the umeclidinium and vilanterol trifenatate as described above can be the only active medicaments in the composition.

A propellant comprising 1,1-difluoroethane (also known as HFA-152a) is also included in the formulation. In certain embodiments, the propellant can further comprise 1,1,1,2,3,3,3-heptafluoropropane (also known as HFA-227 or HFC-227), and/or 1,1,1,2-tetrafluoroethane (also known as HFA-134 or HFC-134), in combination with 1,1-difluoroethane (HFA-152a). In some embodiments the propellant consists essentially of 1,1-difluoroethane (HFA-152a). The propellant also serves as a dispersant for the particles of umeclidinium, such as umeclidinium bromide, and vilanterol trifenatate.

The particles of umeclidinium, such as umeclidinium bromide, and vilanterol trifenatate are not dissolved in the formulation. Instead, the particles of umeclidinium, such as umeclidinium bromide, and vilanterol trifenatate are suspended in the propellant.

In some embodiments the composition consists essentially of umeclidinium, vilanterol trifenatate, and one or more propellants.

In order to facilitate this suspension, additional components can be added to the formulation. One such additional component is ethanol. Another such additional component is a surfactant. Another such additional component is an excipient such as lactose. These additional components are not required unless otherwise specified.

When ethanol is used, it can be employed in relatively low concentrations. On a weight percent basis, the amount of ethanol used, if any, can be no greater than 5, no greater than 4.5, no greater than 4.0, no greater than 3.5, no greater than 3.0, no greater than 2.5, no greater than 2.0, no greater than 1.5, no greater than 1.4, no greater than 1.3, no greater than 1.2, no greater than 1.1, no greater than 1.0, no greater than 0.9, no greater than 0.8, no greater than 0.7, no greater than 0.6, or no greater than 0.5. On a weight percent basis, the amount of ethanol used, if any, can be no less than 0.5, no less than 0.6, no less than 0.7, no less than 0.8, no less than 0.9, no less than 1.0, no less than 1.1, no less than 1.1, no less than 1.2, no less than 1.3, no less than 1.4, no less than 1.5, no less than 2.0, no less than 2.5, no less than 3.0, no less than 3.5, no less than 4 .0, no less than 4.5, or no less than 5.0. Exemplary ranges of ethanol concentration, in those cases when ethanol is included, are from 0.1 wt.% to 5 wt.%, such as from 0.5 wt.% to 4 wt.%. In some cases, an ethanol concentration of 1 wt.% is employed.

One or more surfactants can also be used to facilitate suspension of the particles in the formulation. However, surfactant-free formulations can be advantageous for some purposes, and surfactant is not required unless otherwise specified.

Any pharmaceutically acceptable surfactant can be used. Most such surfactants are suitable for use with an inhaler. Exemplary surfactants include oleic acid, sorbitan monooleate, sorbitan trioleate, soya lecithin, polyethylene glycol, polyvinylpyrrolidone, or combinations thereof. Oleic acid, polyvinylpyrrolidone, or a combination thereof is most common. A combination of polyvinylpyrrolidone and polyethylene glycol is also commonly employed. When polyvinylpyrrolidone is employed, it can have any suitable molecular weight. Examples of suitable weight average molecular weights are from 10 to 100 kilodaltons, and can be from 10 to 50, 10 to 40, 10 to 30 or 10 to 20 kilodaltons. When polyethylene glycol is employed, it can be any suitable grade. PEG 1000 and PEG 300 are most commonly employed.

When used, the surfactant can be present, on a weight percent basis, in an amount no less than 0.0001, no less than 0.01, no less than 0.02, no less than 0.03, no less than 0.04, no less than 0.05, no less than 0.06, no less than 0.07, no less than 0.08, no less than 0.09, no less than 0.10, no less than 0.15, no less than 0.20, no less than 0.25, no less than 0.3, no less than 0.4, no less than 0.5, no less than 0.6, no less than 0.7, no less than 0.8, no less than 0.9, or no less than 1. The surfactant can be present, on a weight percent basis, in an amount no greater than 1, no greater than 0.9, no greater than 0.8, no greater than 0.7, no greater than 0.6, no greater than 0.5, no greater than 0.4, no greater than 0.3, no greater than 0.25, no greater than 0.20, no greater than 0.15, no greater than 0.14, no greater than 0.13, no greater than 0.12, no greater than 0.11, no greater than 0.10, no greater than 0.09, no greater than 0.08, no greater than 0.07, no greater than 0.06, no greater than 0.05, no greater than 0.04, no greater than 0.03, no greater than 0.02, or no greater than 0.01. Concentration ranges can be from 0.0001 wt.% to 1 wt.%, such as 0.001 wt.% to 0.1 wt.%. Particular applications use 0.01 wt.% surfactant.

Particularly, oleic acid can be used in any of the abovementioned concentrations. Particularly, polyvinylpyrrolidone can be used in any of the abovementioned concentrations. Particularly, a combination of polyethylene glycol and polyvinylpyrrolidone can be used in any of the abovementioned concentrations. Particularly, sorbitan trioleate can be used in any of the abovementioned concentrations.

When lactose is used as an excipient, the lactose can be a particulate. In another embodiment, the lactose can micronized. In an additional embodiment, the lactose can be lactose nanoparticles.

The above-described formulations can be used with metered dose inhalers known in the art.

Exemplary metered dose inhalers for the pharmaceutical formulations described herein contain an aerosol canister fitted with a valve. The canister can have any suitable volume. The brimful capacity canister will depend on the volume of the formulation that is used to fill the canister. In exemplary applications, the canister will have a volume from 5 mL to 500 mL, such as, for example 10 mL to 500 mL, 25 mL to 400 mL, 5 mL to 50 mL, 8 mL to 30 mL, 10 mL to 25 mL, or 5 to 20 mL. The canister will often have sufficient volume to contain enough medicament for delivering an appropriate number of doses. The appropriate number of doses is discussed herein. The valve can be affixed, or crimped, onto the canister by way of a cap or ferrule. The cap or ferrule is often made of aluminum or an aluminum alloy, which can be part of the valve assembly. One or more seals can be located between the canister and the ferrule. The seals can be one or more of O-ring seals or gasket seals. The valve can be a metered dose valve. Exemplary valve sizes range from 20 microliters to 100 microliters. Specific valve size that are commonly employed include 25, 50, 60, and 63 microliter valve sizes. The valve may contain a desiccating material, such as nylon. Further, the metered dose inhaler may contain secondary packaging (foil pouch with or without desiccant such as silica gel or suitable molecular sieve) to control the level of water within the formulation.

The container and valve can include an actuator. Most actuators have a patient port, which can be a mouthpiece, for delivering the formulation contained in the canister. The patient port can be configured in a variety of ways depending on the intended destination of the formulation. For example, a patient port designed for administration to the nasal cavities will generally have an upward slope to direct the formulation to the nose. The actuator is most commonly made of plastic material. Exemplary plastic materials for this purpose include at least one of polyethylene and polypropylene. Exemplary MDIs have an actuator with an orifice diameter. Any suitable orifice diameter can be used. Exemplary orifice diameters are from 0.2 mm to 0.65 mm. Exemplary orifice jet length is from 0.5 mm to 1.5 mm. Specific examples include orifice diameters of 0.2 mm, 0.25 mm, 0.3 mm, 0.4 mm, 0.5 mm, or 0.6 mm, any of which can have an orifice jet length of 0.8 mm, 1.0 mm, or 1.5 mm.

A metered dose valve can be present and is often located at least partially within the canister and at least partially in communication with the actuator. Exemplary metered dose valves include a metering chamber that is at least partially defined by an inner valve body through which a valve stem passes. The valve stem can be biased outwardly by a compression spring to be in a sliding sealing engagement with an inner tank seal and outer diaphragm seal. The valve can also include a second valve body in the form of a body emptier. The inner valve body, which is sometimes referred to as the primary valve body, defines, in part, the metering chamber. The second valve body, which is sometimes referred to as the secondary valve body, defines, in part, a pre-metering region (sometimes called a pre-metering chamber) in addition to serving as a bottle emptier. The outer walls of the portion of the metered dose valve that are located within the canister, as well as the inner walls of the canister, defined a formulation chamber for containing the pharmaceutical formulation.

In use, the pharmaceutical formulation passes from the formulation chamber into the metering chamber. In moving to the metering chamber, the formulation can pass into the above-mentioned pre-metering chamber through an annular space between the secondary valve body (or a flange of the secondary valve body) and the primary valve body. Pressing the valve stem towards the interior of the container actuates the valve, which allows the pharmaceutical formulation to pass from the pre-metering chamber through a side hole in the valve stem, through an outlet in the valve stem, to an actuator nozzle, and finally through the patient port to the patient. When the valve stem is released, the pharmaceutical formulation enters the valve, such as to the pre-metering chamber, through an annular space and then travels to the metering chamber.

The pharmaceutical formulation can be placed into the canister by any known method. The two most common methods are cold filling and pressure filling. In a cold filling process, the pharmaceutical formulation is chilled to an appropriate temperature, which can be -40 °C to -60 °C for formulations that use propellant HFA 152a, optionally in combination with HFA 134a and/or HFA 227, and added to the canister. The metered dose valve is subsequently crimped onto the canister. When the canister warms to ambient temperature, the vapor pressure associated with the pharmaceutical formulation increases thereby providing an appropriate pressure within the canister.

In a pressure filling method, the metered dose valve can be first crimped onto the empty canister. Subsequently, the formulation can be added through the valve into the container by way of applied pressure. Alternatively, all the non-volatile components can be first added to the empty canister before crimping the valve onto the canister. The propellant can then be added through the valve into the canister by way of applied pressure.

Upon actuation, exemplary metered dose inhalers that are filled with any one of the formulations described herein can produce a fine particle mass of vilanterol, that is from 5 mcg to 20 mcg per actuation and a fine particle mass of umeclidinium, that is from 5 mcg to 25 mcg per actuation. In particular cases, inhalers, such as metered dose inhalers, produce a fine particle mass of vilanterol that is from 6 mcg to 12 mcg, and a fine particle mass of umeclidinium, that is from 10 mcg to 20 mcg per actuation. In particular cases, inhalers, such as metered dose inhalers, produce a fine particle mass of vilanterol that is from 6 mcg to 12 mcg, and a fine particle mass of umeclidinium, that is from 12 mcg to 18 mcg per actuation.

The fine particle masses discussed above can correspond to a fine particle fraction of vilanterol and of umeclidinium, particularly umeclidinium bromide, that is from 20% to 65%, which can be from 20% to 40% in particular cases, or from 25% to 35% in more particular cases.

Exemplary metered dose inhalers are designed to deliver a specified number of doses of the pharmaceutical formulation. In most cases, the specified number of doses is from 15 to 400, such as from 120 to 250 or such as 15 to 60. One commonly employed metered dose inhaler is designed to provide 120 doses; this can be employed with any of the formulations or inhaler types described herein. Another commonly employed metered dose inhaler is designed to provide 240 doses; this can be employed with any of the formulations or inhaler types described herein. In another embodiment, a metered dose inhaler may provide 30 doses.

The metered dose inhaler can contain a dose counter for counting the number of doses. Suitable dose counters are known in the art, and are described in, for example, U.S. Patent Nos. 8,740,014, 8,479,732, and 8,814,035, and U.S. Patent Application Publication No. 2012/0234317.

One exemplary dose counter, which is described in detail in U.S. Patent No. 8,740,014, has a fixed ratchet element and a trigger element that is constructed and arranged to undergo reciprocal movement coordinated with the reciprocal movement between an actuation element in an inhaler and the dose counter. The reciprocal movement can comprise an outward stroke (outward being with respect to the inhaler) and a return stroke. The return stroke returns the trigger element to the position that it was in prior to the outward stroke. A counter element is also included in this type of dose counter. The counter element is constructed and arranged to undergo a predetermined counting movement each time a dose is dispensed. The counter element is biased towards the fixed ratchet and trigger elements and is capable of counting motion in a direction that is substantially orthogonal to the direction of the reciprocal movement of the trigger element.

The counter element in the above-described dose counter comprises a first region for interacting with the trigger member. The first region comprises at least one inclined surface that is engaged by the trigger member during the outward stroke of the trigger member. This engagement during the outward stroke causes the counter element to undergo a counting motion. The counter element also comprises a second region for interacting with the ratchet member. The second region comprises at least one inclined surface that is engaged by the ratchet element during the return stroke of the trigger element causing the counter element to undergo a further counting motion, thereby completing a counting movement. The counter element is normally in the form of a counter ring, and is advanced partially on the outward stroke of the trigger element, and partially on the return stroke of the trigger element. As the outward stroke of the trigger can correspond to the depression of a valve stem that causes firing of the valve (and, in the case of a metered dose inhaler, also meters the contents) and the return stroke can correspond to the return of the valve stem to its resting position, this dose counter allows for precise counting of doses.

Another suitable dose counter, which is described in detail in U.S. Patent No. 8,479,732, is specially adapted for use with a metered dose inhaler. This dose counter includes a first count indicator having a first indicia bearing surface. The first count indicator is rotatable about a first axis. The dose counter also includes a second count indicator having a second indicia bearing surface. The second count indicator is rotatable about a second axis. The first and second axes are disposed such that they form an obtuse angle. The obtuse angle mentioned above can be any obtuse angle but is advantageously 125 to 145 degrees. The obtuse angle permits the first and second indicia bearing surface to align at a common viewing area to collectively present at least a portion of a medication dosage count. One or both of the first and second indicia bearing surfaces can be marked with digits, such that when viewed together through the viewing area the numbers provide a dose count. For example, one of the first and second indicia bearing surface may have "hundreds" and "tens" place digits, and the other with "ones" place digits, such that when read together the two indicia bearing surfaces provide a number between 000 and 999 that represents the dose count.

Yet another suitable dose counter is described in U.S. Patent Application Publication No. 2012/0234317.

Such a dose counter includes a counter element that undergoes a predetermined counting motion each time a dose is dispensed. The counting motion can be vertical or essentially vertical. A count indicating element is also included. The count indicating element, which undergoes a predetermined count indicating motion each time a dose is dispensed, includes a first region that interacts with the counter element.

The counter element has regions for interacting with the count indicating element. Specifically, the counter element comprises a first region that interacts with a count indicating element. The first region includes at least one surface that it engaged with at least one surface of the first region of the aforementioned count indicating element. The first region of the counter element and the first surface of the count inducing element are disposed such that the count indicating member completes a count indicating motion in coordination with the counting motion of the counter element, during and induced by the movement of the counter element, the count inducing element undergoes a rotational or essentially rotational movement. In practice, the first region of the counter element or the counter indicating element can comprise, for example, one or more channels. A first region of the other element can comprise one or more protrusions adapted to engage with said one or more channels.

Yet another dose counter is described in U.S. Patent No. 8,814,035.

Such a dose counter is specially adapted for use with an inhaler with a reciprocal actuator operating along a first axis. The dose counter includes an indicator element that is rotatable about a second axis. The indicator element is adapted to undergo one or more predetermined count-indicating motions when one or more doses are dispensed. The second axis is at an obtuse angle with respect to the first axis. The dose counter also contains a worm rotatable about a worm axis. The worm is adapted to drive the indicator element. It may do this, for example, by containing a region that interacts with and enmeshes with a region of the indicator element. The worm axis and the second axis do not intersect and are not aligned in a perpendicular manner. The worm axis is also, in most cases, not disposed in coaxial alignment with the first axis. However, the first and second axes may intersect.

At least one of the various internal components of an inhaler, such as a metered dose inhaler, as described herein, such as one or more of the canister, valve, gaskets, seals, ferrule, or O-rings, can be coated with one or more coatings. Some of these coatings provide a low surface energy. Such coatings are not required because they are not necessary for the successful operation of all inhalers.

Some coatings that can be used are described in U.S. Patent Nos. 8,414,956 and 8,815,325 and United States Patent Application Publication No. 2012/0097159.

Other coatings, such as fluorinated ethylene propylene resins, or FEP, and FCP, PTFE are also suitable. FEP is particularly suitable for use in coating canisters.

A first acceptable coating can be provided by the following method:
a) providing one or more component of the inhaler, such as the metered dose inhaler,
b) providing a primer composition comprising a silane having two or more reactive silane groups separated by an organic linker group,
c) providing a coating composition comprising an at least partially fluorinated compound,
d) applying the primer composition to at least a portion of the surface of the component,
e) applying the coating composition to the portion of the surface of the component after application of the primer composition.

The at least partially fluorinated compound will usually comprise one or more reactive functional groups, with the or each one reactive functional group usually being a reactive silane group, for example a hydrolysable silane group or a hydroxysilane group. Such reactive silane groups allow reaction of the partially fluorinated compound with one or more of the reactive silane groups of the primer. Often such reaction will be a condensation reaction.

One exemplary silane that can be used has the formula

X₃₋ₘ(R¹)ₘSi - Q - Si(R²)ₖX₃₋ₖ

wherein R¹ and R² are independently selected univalent groups, X is a hydrolysable or hydroxy group, m and k are independently 0, 1, or 2 and Q is a divalent organic linking group.

Useful examples of such silanes include one or a mixture of two or more of 1,2-bis(trialkoxysilyl) ethane, 1,6-bis(trialkoxysilyl) hexane, 1,8-bis(trialkoxysilyl) octane, 1,4-bis(trialkoxysilylethyl)benzene, bis(trialkoxysilyl)itaconate, and 4,4'-bis(trialkoxysilyl)-1,1'-diphenyl, wherein any trialkoxy group may be independently trimethoxy or triethoxy.

The coating solvent usually comprises an alcohol or a hydrofluoroether.

If the coating solvent is an alcohol, preferred alcohols are C₁ to C₄ alcohols, in particular, an alcohol selected from ethanol, n-propanol, or isopropanol or a mixture of two or more of these alcohols.

If the coating solvent is an hydrofluoroether, it is preferred if the coating solvent comprises a C₄ to C₁₀ hydrofluoroether. Generally, the hydrofluoroether will be of formula

C_{g}F_{2g+1}OCₕH₂ₕ₊₁

wherein g is 2, 3, 4, 5, or 6 and h is 1, 2, 3 or 4. Examples of suitable hydrofluoroethers include those selected from the group consisting of methyl heptafluoropropylether, ethyl heptafluoropropylether, methyl nonafluorobutylether, ethyl nonafluorobutylether and mixtures thereof.

The polyfluoropolyether silane can be of the formula

R*^{f}*Q¹ᵥ[Q²_{w}-[C(R⁴)₂-Si(X)₃₋ₓ(R⁵)ₓ]_{y}]_{z}

wherein:
R*^{f}* is a polyfluoropolyether moiety;
Q¹ is a trivalent linking group;
each Q² is an independently selected organic divalent or trivalent linking group;
each R⁴ is independently hydrogen or a C₁₋₄ alkyl group;
each X is independently a hydrolysable or hydroxyl group;
R⁵ is a C₁₋₈ alkyl or phenyl group;
v and w are independently 0 or 1, x is 0 or 1 or 2; y is 1 or 2; and z is 2, 3, or 4.

The polyfluoropolyether moiety R*^{f}* can comprise perfluorinated repeating units selected from the group consisting of -(CₙF₂ₙO)-, -(CF(Z)O)-, -(CF(Z)CₙF₂ₙO)-, -(CₙF₂ₙCF(Z)O)-, -(CF₂CF(Z)O)-, and combinations thereof; wherein n is an integer from 1 to 6 and Z is a perfluoroalkyl group, an oxygen-containing perfluoroalkyl group, a perfluoroalkoxy group, or an oxygen-substituted perfluoroalkoxy group, each of which can be linear, branched, or cyclic, and have 1 to 5 carbon atoms and up to 4 oxygen atoms when oxygen-containing or oxygen-substituted and wherein for repeating units including Z the number of carbon atoms in sequence is at most 6. In particular, n can be an integer from 1 to 4, more particularly from 1 to 3. For repeating units including Z the number of carbon atoms in sequence may be at most four, more particularly at most 3. Usually, n is 1 or 2 and Z is an -CF₃ group, more wherein z is 2, and R*^{f}* is selected from the group consisting of -CF₂(CF₂)ₘ(C₂F₄O)ₚCF₂-, -CF(CF₃)O(CF(CF₃)CF₂O)CF(CF₃)-, -CF₂O(C₂F₄O)ₚCF₂-, -(CF₂)₃O(C₄F_{g}O)ₚ(CF₂)₃-, -CF(CF₃)-(OCF₂CF(CF₃))ₚO-CₜF₂ₜ-O(CF(CF₃)CF₂O)ₚCF(CF₃)-, wherein t is 2, 3 or 4 and wherein m is 1 to 50, and p is 3 to 40.

A cross-linking agent can be included. Exemplary cross-linking agents include tetramethoxysilane; tetraethoxysilane; tetrapropoxysilane; tetrabutoxysilane; methyl triethoxysilane; dimethyldiethoxysilane; octadecyltriethoxysilane; 3-glycidoxy-propyltrimethoxysilane; 3-glycidoxy-propyltriethoxysilane; 3-aminopropyl-trimethoxysilane; 3-aminopropyl-triethoxysilane; bis(3-trimethoxysilylpropyl) amine; 3-aminopropyl tri(methoxyethoxyethoxy) silane; N ( 2-aminoethyl)3-aminopropyltrimethoxysilane; bis ( 3-trimethoxysilylpropyl) ethylenediamine; 3-mercaptopropyltrimethoxysilane; 3-mercaptopropyltriethoxysilane; 3-trimethoxysilyl-propylmethacrylate; 3-triethoxysilypropylmethacrylate; bis(trimethoxysilyl) itaconate; allyltriethoxysilane; allyltrimethoxysilane; 3-(N-allylamino)propyltrimethoxysilane; vinyltrimethoxysilane; vinyltriethoxysilane; and mixtures thereof.

The component to be coated can be pre-treated before coating, such as by cleaning. Cleaning can be by way of a solvent, such as a hydrofluoroether, e.g. HFE72DE, or an azeotropic mixture of about 70%w/w trans-dichloroethylene; 30% w/w of a mixture of methyl and ethyl nonafluorobutyl and nonafluoroisobutyl ethers.

The above-described first acceptable coating is particularly useful for coating valves components, including one or more of valve stems, bottle emptiers, springs, ferrule and tanks. This coating system can be used with any type of inhaler and any formulation described herein.

In embodiments the pharmaceutical performance of a MDI of the present invention is controlled so that it is similar to the pharmaceutical performance of a reference inhaler. For example, in embodiments the pharmaceutical performance of a MDI of the present invention is similar to that of Anoro ^{®} Ellipta ^{®} 62.5/25, a dry powder inhalation product that contains individual doses of 74.2 micrograms of umeclidinium bromide (equivalent to 62.5 micrograms of umeclidinium) and 25 micrograms of vilanterol. The dose of vilanterol in Anoro ^{®} Ellipta ^{®} 62.5/25 is given as a base equivalent, i.e., the dose is 25 micrograms of vilanterol base present in the form of vilanterol trifenatate. This is likewise true for the respective vilanterol doses of the other Anoro products described below. In embodiments the pharmaceutical performance of a MDI of the present disclosure is similar to that of Anoro ^{®} Ellipta ^{®} 55/22, a dry powder inhalation product that delivers a nominal dose of 65 micrograms of umeclidinium bromide (equivalent to 55 micrograms umeclidinium) and 22 micrograms of vilanterol per inhalation.

Similar pharmaceutical performance may be evaluated by either in vitro or in vivo test methods.

Suitable in vitro test methods include, but are not limited to, single actuation content and aerodynamic particle size distribution. Single actuation content may be measured at beginning, middle, and/or end of life for an MDI using a flow rate of 28.3 L/min. U.S. Pharmacopeia (USP) <601> Apparatus A or other appropriate apparatus may be used. Aerodynamic particle size distribution may be measured at beginning, middle, and/or end of life using a flow rate of 28.3 L/min. The USP <601> Apparatus 1, Apparatus 6, or other appropriate apparatus may be used. Single actuation content may be further analyzed to determine fine particle mass (FPM) and/or impactor stage mass (ISM). Aerodynamic particle size distribution may be further analyzed to determine mass median aerodynamic diameter (MMAD).

Suitable in vivo test methods include, but are not limited to, pharmacokinetic (PK) bioequivalence studies and clinical pharmacodynamic bioequivalence studies. One of ordinary skill in the art will understand the appropriate parameters and ranges of performance that are required to establish bioequivalence. An exemplary PK bioequivalence study will be considered to have established bioequivalence if the area under the curve (AUC) and the Cₘₐₓ (maximum concentration) of active and/or active metabolite in the plasma has a 90% confidence interval for the geometric mean of the ratio between test and reference articles that falls within limits of 80 to 125%. An exemplary clinical pharmacodynamic bioequivalence study will establish bioequivalence if one or more clinical measures of lung function, such as FEV1, have a 90% confidence interval for the mean of the ratio between test and reference articles that falls within limits of 80 to 125%.

### EXAMPLES

1,1-difluoroethane (HFA-152a) was obtained from Mexichem (Runcorn, UK). Vilanterol trifenatate was obtained from Hovione (Portugal). Umeclidinium bromide was obtained from Hovione (Portugal).

### Example 1

Metered dose inhalers (MDIs) according to an embodiment of the present disclosure are prepared using 16 mL aluminum canisters coated with FEP (IntraPac International, Mooresville, NC, USA), 63 microliter 3M retention type valves with a PBT (polybutylene terephthalate) stem and EPDM (ethylene-propylene diene terpolymer elastomer) diaphragm seals (3M Corporation), and 0.25 mm exit orifice diameter 3M Mk6 actuators (Oechsler, Ansbach, Germany) fitted with an integrated dose counter. The valves are coated with a fluoropolymer coating according to the general process described in Example 2 of U.S. Patent Application Publication 2017/0152396 A1, Jinks et al. Vilanterol trifenatate is particle size reduced to provide a mass median diameter (MMD) of about 1.5 microns. Umeclidinium bromide is particle size reduced to provide a mass median diameter (MMD) of about 2.0 microns. The canisters are cold filled with a suspension formulation having 0.1294% umeclidinium bromide, 0.0698% vilanterol trifenatate, and 99.8008% HFA-152a. The bulk formulation for cold filling individual canisters are prepared by combining umeclidinium bromide and vilanterol trifenatate with the HFA-152a propellant in a vessel chilled to less than -40 °C. The suspension is mixed until the suspension is adequately dispersed.

## Claims

1. A composition comprising:
particulate umeclidinium or a pharmaceutically acceptable salt or solvate thereof;
particulate vilanterol trifenatate; and
a propellant comprising 1,1-difluoroethane (HFA-152a);
wherein the particulate umeclidinium or the pharmaceutically acceptable salt or solvate thereof and the particulate vilanterol trifenatate are suspended in the propellant.

2. The composition of claim 1, wherein umeclidinium or the pharmaceutically acceptable salts or solvates thereof and vilanterol trifenatate are the only actives in the composition.

3. The composition of any previous claim, wherein the umeclidinium or a pharmaceutically acceptable salt or solvate thereof is umeclidinium bromide.

4. The composition of any previous claim, wherein the propellant consists essentially of 1,1-difluoroethane (HFA-152a).

5. The composition of any previous claim, wherein the canister size of the umeclidinium is between about 2 micrometers and 4 micrometers.

6. The composition of any previous claim, wherein the canister size of the vilanterol is between about 1 micrometer and 2 micrometers.

7. The composition of any previous claim, wherein the concentration of the umeclidinium is between about 0.5 mg/g and 5.0 mg/g of the composition.

8. The composition of any previous claim, wherein the concentration of the vilanterol is between about 0.2 mg/g and 2.6 mg/g of the composition.

9. An aerosol canister comprising a composition of any preceding claim.

10. The aerosol canister of claim 9 comprising at least one surface having a primer composition comprising a silane having two or more reactive silane groups separated by an organic linker group disposed thereon, wherein the primer composition has a coating composition comprising an at least partially fluorinated compound disposed thereon.

11. The aerosol canister of claim 10, wherein the at least partially fluorinated compound is a polyfluoropolyether silane.

12. The aerosol canister of claims 10 or 11, wherein the at least one surface is at least a portion of a valve surface.

13. An inhaler comprising the composition of any one of claims 1 to 8 or the aerosol canister of any one of claims 9 to 12.

14. The inhaler of claim 13, which is a metered dose inhaler, preferably a pressurized metered dose inhaler.

## Patentansprüche

1. Eine Zusammensetzung, umfassend:
teilchenförmiges Umeclidinium oder ein pharmazeutisch verträgliches Salz oder Solvat davon;
teilchenförmiges Vilanteroltrifenatat; und
ein Treibmittel, umfassend 1,1-Difluorethan (HFA-152a);
wobei das teilchenförmige Umeclidinium oder das pharmazeutisch verträgliche Salz oder Solvat davon und das teilchenförmige Vilanteroltrifenatat in dem Treibmittel suspendiert sind.

2. Die Zusammensetzung nach Anspruch 1, wobei Umeclidinium oder die pharmazeutisch verträglichen Salze oder Solvate davon und Vilanteroltrifenatat die einzigen Wirkstoffe in der Zusammensetzung sind.

3. Die Zusammensetzung nach einem der vorhergehenden Ansprüche, wobei das Umeclidinium oder ein pharmazeutisch verträgliches Salz oder Solvat davon Umeclidiniumbromid ist.

4. Die Zusammensetzung nach einem der vorhergehenden Ansprüche, wobei das Treibmittel im Wesentlichen aus 1,1-Difluorethan (HFA-152a) besteht.

5. Die Zusammensetzung nach einem der vorhergehenden Ansprüche, wobei die Behältergröße des Umeclidiniums zwischen etwa 2 Mikrometern und 4 Mikrometern liegt.

6. Die Zusammensetzung nach einem der vorhergehenden Ansprüche, wobei die Behältergröße des Vilanterols zwischen etwa 1 Mikrometer und 2 Mikrometern liegt.

7. Die Zusammensetzung nach einem der vorhergehenden Ansprüche, wobei die Konzentration des Umeclidiniums zwischen etwa 0,5 mg/g und 5,0 mg/g der Zusammensetzung beträgt.

8. Die Zusammensetzung nach einem der vorhergehenden Ansprüche, wobei die Konzentration des Vilanterols zwischen etwa 0,2 mg/g und 2,6 mg/g der Zusammensetzung beträgt.

9. Ein Aerosolbehälter, umfassend eine Zusammensetzung nach einem der vorhergehenden Ansprüche.

10. Der Aerosolbehälter nach Anspruch 9, umfassend mindestens eine Oberfläche mit einer darauf befindlichen Grundierungszusammensetzung, die ein Silan mit zwei oder mehreren reaktiven Silangruppen, die durch eine organische Linkergruppe getrennt sind, umfasst, wobei die Grundierungszusammensetzung eine Beschichtungszusammensetzung aufweist, die eine darauf befindliche mindestens teilweise fluorierte Verbindung umfasst.

11. Der Aerosolbehälter nach Anspruch 10, wobei die mindestens teilweise fluorierte Verbindung ein Polyfluorpolyethersilan ist.

12. Der Aerosolbehälter nach Anspruch 10 oder 11, wobei die mindestens eine Oberfläche mindestens ein Teil einer Ventiloberfläche ist.

13. Ein Inhalator, umfassend die Zusammensetzung nach einem der Ansprüche 1 bis 8 oder den Aerosolbehälter nach einem der Ansprüche 9 bis 12.

14. Der Inhalator nach Anspruch 13, bei welchem es sich um einen Dosierinhalator, vorzugsweise einen unter Druck stehenden Dosierinhalator, handelt.

## Revendications

1. Composition comprenant :
de l'uméclidinium particulaire ou un solvate ou sel pharmaceutiquement acceptable de celui-ci ;
du trifénatate de vilantérol particulaire ; et
un propulseur comprenant du 1,1-difluoroéthane (HFA-152a) ;
dans laquelle l'uméclidinium particulaire ou le solvate ou sel pharmaceutiquement acceptable de celui-ci et le trifénatate de vilantérol particulaire sont en suspension dans le propulseur.

2. Composition selon la revendication 1, dans laquelle l'uméclidinium ou les solvates ou sels pharmaceutiquement acceptables de celui-ci et le trifénatate de vilantérol sont les seuls principes actifs dans la composition.

3. Composition selon l'une quelconque des revendications précédentes, dans laquelle l'uméclidinium ou un solvate ou sel pharmaceutiquement acceptable de celui-ci est le bromure d'uméclidinium.

4. Composition selon l'une quelconque des revendications précédentes, dans laquelle le propulseur consiste essentiellement en 1,1-difluoroéthane (HFA-152a).

5. Composition selon l'une quelconque des revendications précédentes, dans laquelle la taille de la cartouche de l'uméclidinium est comprise entre environ 2 micromètres et 4 micromètres.

6. Composition selon l'une quelconque des revendications précédentes, dans laquelle la taille de la cartouche du vilantérol est comprise entre environ 1 micromètre et 2 micromètres.

7. Composition selon l'une quelconque des revendications précédentes, dans laquelle la concentration de l'uméclidinium est comprise entre environ 0,5 mg/g et 5,0 mg/g de la composition.

8. Composition selon l'une quelconque des revendications précédentes, dans laquelle la concentration du vilantérol est comprise entre environ 0,2 mg/g et 2,6 mg/g de la composition.

9. Cartouche aérosol comprenant une composition de l'une quelconque des revendications précédentes.

10. Cartouche aérosol selon la revendication 9, comprenant au moins une surface ayant une composition d'apprêt comprenant un silane ayant deux ou plus de deux groupes silane réactifs séparés par un groupe lieur organique disposé sur celle-ci, dans laquelle la composition d'apprêt a une composition de revêtement comprenant un composé au moins partiellement fluoré disposée sur celle-ci.

11. Cartouche aérosol selon la revendication 10, dans laquelle le composé au moins partiellement fluoré est un polyfluoro-polyéther-silane.

12. Cartouche aérosol selon la revendication 10 ou 11, dans laquelle la au moins une surface est au moins une portion d'une surface de vanne.

13. Inhalateur comprenant la composition de l'une quelconque des revendications 1 à 8 ou la cartouche aérosol de l'une quelconque des revendications 9 à 12.

14. Inhalateur selon la revendication 13, qui est un inhalateur doseur, de préférence un inhalateur doseur pressurisé.
